# EUROPEAN PATENT APPLICATION

(11) **EP 1 944 058 A1**
(43) Date of publication of application: **16.07.2008**
(21) Application number: 06811665.6
(22) Date of filing: 12.10.2006
(51) Int. Cl.: A61N 1/30, A61K 9/70, A61K 47/36

(54) **IONTOPHORESIS APPARATUS STICKING TO MUCOSA**

(30) Priority: 12.10.2005 JP 2005297745
(71) Applicant: TTI ellebeau, Inc., Shinagawa-ku Tokyo (JP)
(72) Inventor: AKIYAMA, Hidero c/o TTI ellebeau, Inc., Shibuya-ku, Tokyo, 1500022 (JP); NAKAYAMA, Mizuo c/o TTI ellebeau, Inc., Shibuya-ku, Tokyo, 1500022 (JP); MATSUMURA, Takehiko c/o TTI ellebeau, Inc., Shibuya-ku, Tokyo, 1500022 (JP); MATSUMURA, Akihiko c/o TTI ellebeau, Inc., Shibuya-ku, Tokyo, 1500022 (JP)
(74) Representative: Liesegang, Eva
(86) International application number: PCT/JP2006/320368
(87) International publication number: WO 2007/043605

(57) **Abstract**

The present invention relates to an iontophoresis device comprising: an electric power source device; a first electrode assembly for administering an ionic drug to a living body through a mucous membrane by releasing the ionic drug by iontophoresis, the first electrode assembly being connected to the electric power source device; and a second electrode assembly as a counter electrode of the first electrode assembly, wherein the first electrode assembly comprises an adhesive portion for bringing the mucous membrane and the first electrode assembly into contact with each other, the adhesive portion being placed on at least a part of an end face portion; and the adhesive portion is composed of a member that exhibits adhesiveness owing to absorption of an aqueous medium.

## Description

### CROSS-REFERENCE TO RELATED APPLICATIONS

This application claims the benefit of priority on the basis of the prior Japanese Patent Application No. 2005-297745 (filed on date: October 12, 2005), the entire disclosure of which is incorporated herein by reference.

### BACKGROUND OF THE TNVENTTON

### Field of Invention

The present invention relates to a technique for transdermally administering various ionic drugs by means of iontophoresis (transdermal drug delivery). More specifically, the present invention relates to an iontophoresis device useful in efficiently and stably administering an ionic drug into a mucous membrane containing water.

### Background Art

A method of introducing (permeating) an ionic drug placed on the surface of a skin or mucous membrane (hereinafter, merely referred to as "skin") of a predetermined site of a living body into the body through the skin by giving the skin an electromotive force sufficient to drive such an ionic drug is called iontophoresis (iontophorese, ion introduction method, ion permeation therapy) (see, for example, JP 63-35266 A).

For example, positively charged ions are driven (transported) into the skin on the side of an anode (positive electrode) in an electric system of an iontophoresis device. On the other hand, negatively charged ions are driven (transported) into the skin on the side of a cathode (negative electrode) in the electric system of the iontophoresis device.

Conventionally, a large number of such iontophoresis devices as described above have been proposed (see, for example, JP 63-35266 A, JP 04-297277 A, JP 2000-229128 A, JP 2000-229129 A, JP 2000-237327 A, JP 2000-237328 A and WO 03/037425 A1).
Such conventional iontophoresis device as described above is requested to efficiently and stably administer an ionic drug, to thereby secure a sufficient therapeutic effect. However, an iontophoresis device is hardly fixed to a biological organ containing water in its surface typified by a mucous membrane, so the efficiency of the administration of an ionic drug may be significantly affected. Therefore, enabling the efficient and stable administration of an ionic drug into a biological organ containing water in its surface typified by a mucous membrane by means of an iontophoresis device is an important problem in securing a sufficient therapeutic effect.

### SUMMARY OF THE INVENTION

The present invention has been made in view of the above-described problems of the prior art, and an object of the present invention is to provide an iontophoresis device enabling the efficient and stable administration of an ionic drug into a mucous membrane containing water in its surface.

In order to solve the above-described problems, an iontophoresis device comprises: an electric power source device; a first electrode assembly for administering an ionic drug to a living body through a mucous membrane by releasing the ionic drug by iontophoresis, the first electrode assembly being connected to the electric power source device; and a second electrode assembly as a counter electrode of the first electrode assembly, wherein the first electrode assembly comprises an adhesive portion for bringing the mucous membrane and the first electrode assembly into close contact with each other, the adhesive portion being placed on at least a part of an end face portion; and the adhesive portion is composed of a member that exhibits adhesiveness owing to absorption of an aqueous medium.

In another preferred aspect according to the present invention, an iontophoresis device comprises: an electric power source device; a first electrode assembly for administering an ionic drug to a living body through the mucous membrane by releasing the ionic drug by iontophoresis, the first electrode assembly being connected to the electric power source device; and a second electrode assembly as a counter electrode of the first electrode assembly, wherein: the second electrode assembly surrounds an outer circumferential portion of the first electrode assembly; the iontophoresis device comprises an adhesive portion for bringing the mucous membrane and the iontophoresis device into close contact with each other, the adhesive portion surrounding an outer circumferential portion of the second electrode assembly; and the adhesive portion is composed of a member that exhibits adhesiveness owing to absorption of an aqueous medium.

A method of operating the iontophoresis device according to the present invention comprises at least: placing the first electrode assembly and the second electrode assembly on a mucous membrane, respectively; energizing the iontophoresis device with the electric power source device; and allowing the first electrode assembly to release the ionic drug.

As described above, the iontophoresis device according to the present invention is constituted by arranging an adhesive portion for bringing a mucous membrane and an electrode assembly into close contact with each other. As a result, an ionic drug can be efficiently and stably administered into a mucous membrane containing water in its surface.

### BRIEF DESCRIPTION OF THE DRAWINGS

Fig. 1 is a view showing the outline of an iontophoresis device according to an embodiment of the present invention.
Fig. 2 is a perspective view showing the back face side of an electrode assembly in the present invention.
Fig. 3 is a view showing the outline of an iontophoresis device according to another embodiment of the present invention.
Fig. 4 is a perspective view showing the back face side of the iontophoresis device according to another embodiment of the present invention.

### DETAILED DESCRIPTION OF THE INVENTION

As described above, according to one aspect of the present invention, the iontophoresis device comprises: an electric power source device; a first electrode assembly for administering an ionic drug by releasing the ionic drug by iontophoresis to a living body through a mucous membrane, the first electrode assembly being connected to the electric power source device; and a second electrode assembly as a counter electrode of the first electrode assembly, wherein the first electrode assembly has an adhesive portion for bringing the mucous membrane and the first electrode assembly into contact with each other, the adhesive portion being placed on at least a part of an end face portion; and the adhesive portion is composed of a member that exhibits adhesiveness owing to absorption of an aqueous medium.

Hereinafter, the present invention will be described on the basis of a preferred embodiment shown in the drawings.

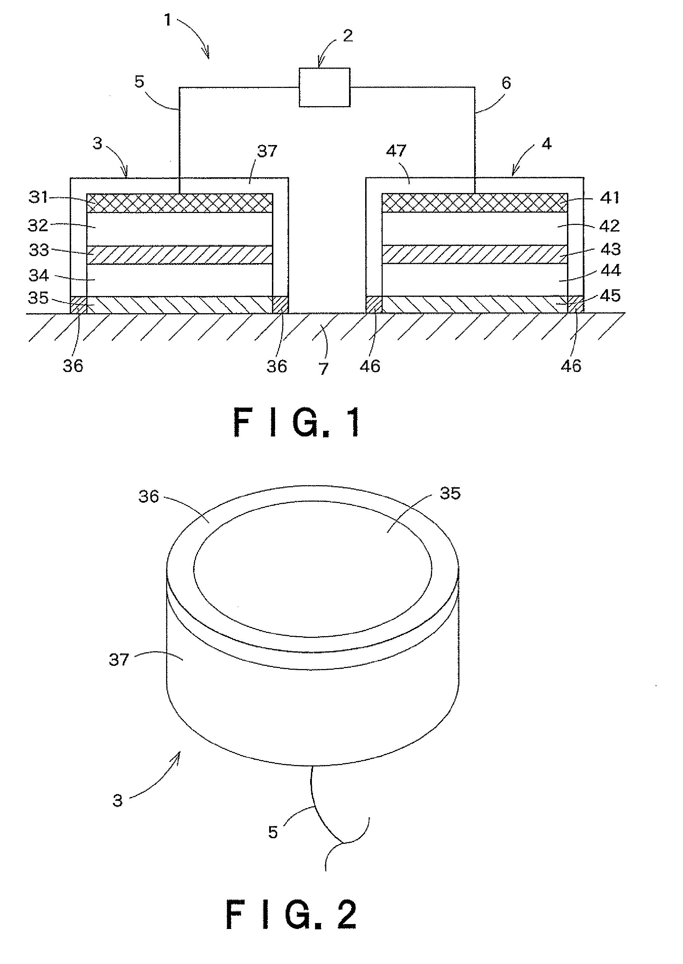

Fig. 1 shows a state where an iontophoresis device 1 is arranged on a mucous membrane 7 containing water in its surface. The iontophoresis device 1 comprises: an electric power source device 2; a first electrode assembly 3 for administering an ionic drug by releasing the ionic drug by iontophoresis to a living body through the mucous membrane, the first electrode assembly 3 being connected to the electric power source device 2; and a second electrode assembly 4 as a counter electrode of the first electrode assembly. Further, in the iontophoresis device 1, the first electrode assembly 3 comprises: an electrode 31 connected to a side of the electric power source device 2 having the same polarity as that of a drug component of the ionic drug via a cord 5; an electrolyte solution holding portion 32 impregnated with the electrolyte solution, the electrolyte solution holding portion 32 being placed adjacent to the electrode 31; an ion exchange membrane 33 which is selectively permeable to an ion having polarity opposite to that of a charged ion of the ionic drug, the ion exchange membrane 33 being placed adjacent to the electrolyte solution holding portion 32; a drug solution holding portion 34 comprising the ionic drug impregnated with the ionic drug, the drug solution holding portion 34 being placed adjacent to the ion exchange membrane 33; an ion exchange membrane 35 which is selectively permeable to an ion having the same polarity as that of the charged ion of the ionic drug, the ion exchange membrane 35 being placed adjacent to the drug solution holding portion 34; and an adhesive portion 36 for bringing the mucous membrane 7 and the first electrode assembly 3 into contact with each other, the adhesive portion 36 being placed on at least a part of an end face portion. Those components are housed in a cover or container 37 constituted by a material such as a resin film or a plastic.

In addition, the second electrode assembly 4 comprises: an electrode 41 having a polarity opposite to that of the electrode 31 in the first electrode assembly 3, the electrode 41 being connected to the electric power source device 2 via a cord 6; an electrolyte solution holding portion 42 impregnated with the electrolyte solution, the electrolyte solution holding portion 42 being placed adjacent to the electrode 41; an ion exchange membrane 43 which is selectively permeable to an ion having the same polarity as that of a charged ion of the ionic drug, the ion exchange membrane 43 being placed adjacent to the electrolyte solution holding portion 42; an electrolyte solution holding portion 44 impregnated with the electrolyte solution, the electrolyte solution holding portion 44 being placed adjacent to the ion exchange membrane 43; an ion exchange membrane 45 which is selectively permeable to an ion having polarity opposite to that of the charged ion of the ionic drug, the ion exchange membrane 45 being placed adjacent to the electrolyte solution holding portion 44; and an adhesive portion 46 for bringing the mucous membrane 7 and the second electrode assembly 4 into contact with each other, the adhesive portion 46 being placed on at least a part of an end face portion. Those components are housed in a cover or container 47 constituted by a material such as a resin film or a plastic.

The adhesive portion (36 or 46) in the present invention is composed of a member that exhibits adhesiveness owing to absorption of an aqueous medium such as water. A release sheet (not shown) may be stuck to the outer surface of the adhesive portion. The sheet is peeled at the time of use.

In another preferred embodiment, the first electrode assembly is as described above, and the second electrode assembly comprises: an electrode having a polarity opposite to that of the electrode 31 in the first electrode assembly 3; an electrolyte solution holding portion impregnated with the electrolyte solution, the electrolyte solution holding portion being placed adjacent to the electrode; an ion exchange membrane which is selectively permeable to an ion having polarity opposite to that of the charged ion of the ionic drug, the ion exchange membrane being placed adjacent to the electrolyte solution holding portion; and an adhesive portion for bringing the mucous membrane 7 and the second electrode assembly 4 into contact with each other, the adhesive portion being arranged on at least a part of an end face portion.

Fig. 2 is a perspective view showing the back face side of the first electrode assembly 3. The adhesive portion 36 is placed on the outer circumferential portion of the ion exchange membrane 35 on the end face portion of the first electrode assembly 3. As in the case of the first electrode assembly 3 shown in Fig. 2, the adhesive portion 46 is preferably placed on the outer circumferential portion of the ion exchange membrane 45 on the end face portion of the second electrode assembly 4. However, the arrangement of each adhesive portion can be appropriately changed to the extent that the release of an ionic drug is not inhibited. In the embodiment shown in Fig. 1, each of both electrode assemblies has an adhesive portion. In some embodiments, however, an iontophoresis device can be constituted in such a manner that only the first electrode assembly has an adhesive portion.

In the iontophoresis device 1 shown in Fig. 1, the first electrode assembly 3 and the second electrode assembly 4 are arranged on the mucous membrane 7, energization is performed by means of the electric power source device 2, and an ionic drug is administered from the first electrode assembly 3 to a living body through the mucous membrane. At this time, the ionic drug is efficiently and stably released because the first electrode assembly 3 closely contacts with the mucous membrane via the adhesive portion even when the mucous membrane contains water. In addition, the electrode assembly (3 or 4) in the present invention can prevent damage to the mucous membrane based on an electrochemical reaction even when the assembly closely contacts with the mucous membrane because the assembly has such constitution as described above. Therefore, the ionic drug can be safely administered.

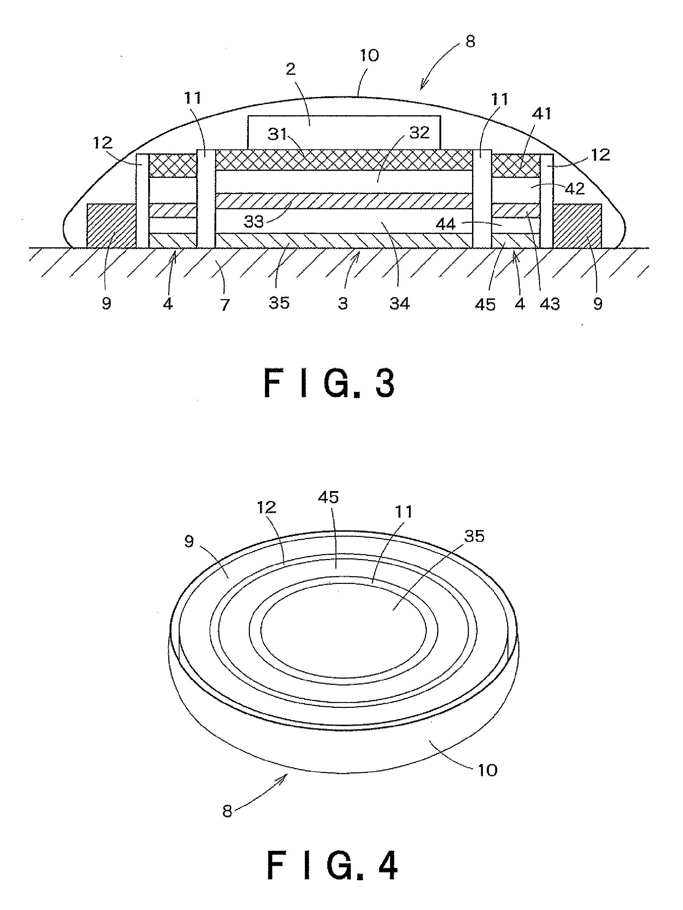

Another preferred example of the iontophoresis device according to the present invention will be described with reference to Fig. 3.
Fig. 3 shows a state where an iontophoresis device 8 constituted integrally is arranged on a mucous membrane 7 containing water in its surface. The iontophoresis device 8 comprises: the electric power source device 2; the first electrode assembly 3 for administering an ionic drug by releasing the ionic drug by iontophoresis to a living body through the mucous membrane, the first electrode assembly 3 being connected to the electric power source device 2; the second electrode assembly 4 as a counter electrode of the first electrode assembly 3; and an adhesive portion 9 for bringing the mucous membrane 7 and the iontophoresis device 8 into close contact with each other. The whole components are housed in a cover or container 10 constituted by a waterproof material such as a resin film or a plastic. In addition, in the iontophoresis device 8, the second electrode assembly 4 surrounds the outer circumferential portion of the first electrode assembly 3, and the adhesive portion 9 surrounds the outer circumferential portion of the second electrode assembly 4. The iontophoresis device 8 further comprises: a first insulating portion 11 placed between the first electrode assembly 3 and the second electrode assembly 4; and a second insulating portion 12 placed between the second electrode assembly 4 and the adhesive portion 9.

As in the case of Fig.1, the adhesive portion 9 is composed of a member that exhibits adhesiveness owing to absorption of an aqueous medium such as water. A release sheet (not shown) may be stuck to the outer surface of the adhesive portion. The sheet is peeled at the time of use.

The first electrode assembly 3 and the second electrode assembly 4 surrounding the outer circumferential portion of the assembly 3 each have substantially the same constitution as that in the case of Fig. 1. That is, the first electrode assembly 3 comprises: the electrode 31 connected to the same polarity of the electric power source device 2 as that of a drug component of the ionic drug; the electrolyte solution holding portion 32 impregnated with the electrolyte solution, the electrolyte solution holding portion 32 being placed adjacent to the electrode 31; the ion exchange membrane 33 which is permeable to an ion having polarity opposite to that of a charged ion of the ionic drug, the ion exchange membrane 33 being placed adjacent to the electrolyte solution holding portion 32; the drug solution holding portion 34 impregnated with the ionic drug, the drug solution holding portion 34 being placed adjacent to the ion exchange membrane 33; and the ion exchange membrane 35 which is selectively permeable to an ion having the same polarity as that of the charged ion of the ionic drug, the ion exchange membrane 35 being placed adjacent to the drug solution holding portion 34.

Meanwhile, the second electrode assembly 4 surrounding the outer circumferential portion of the first electrode assembly 3 comprises: an electrode 41 having a polarity opposite to that of the electrode 31 in the first electrode assembly 3, the electrode 41 being connected to the electric power source device 2 via a cord (not shown); an electrolyte solution holding portion 42 impregnated with the electrolyte solution, the electrolyte solution holding portion 42 being placed adjacent to the electrode 41; an ion exchange membrane 43 which is selectively permeable to an ion having the same polarity as that of the charged ion of the ionic drug, the ion exchange membrane 43 being placed adjacent to the electrolyte solution holding portion 42; an electrolyte solution holding portion 44 impregnated with the electrolyte solution, the electrolyte solution holding portion 44 being placed adjacent to the ion exchange membrane 43; and an ion exchange membrane 45 which is selectively permeable to an ion having polarity opposite to that of the charged ion of the ionic drug, the ion exchange membrane 45 being placed adjacent to the electrolyte solution holding portion 44.

In another preferred embodiment, the second electrode assembly surrounding the first electrode assembly 3 may comprise: an electrode having a polarity opposite to that of the electrode 31 in the first electrode assembly 3; an electrolyte solution holding portion impregnated with the electrolyte solution, the electrolyte solution holding portion being placed adjacent to the electrode; and an ion exchange membrane which is selectively permeable to an ion having polarity opposite to that of the charged ion of the ionic drug, the ion exchange membrane being placed adjacent to the electrolyte solution holding portion.

Fig. 4 is a perspective view showing the back face side of the iontophoresis device 8. In Fig. 4, the ion exchange membrane 45 in the second electrode assembly 4 surrounds the outer circumferential portion of the ion exchange membrane 35 of the first electrode assembly 3, and the adhesive portion 9 surrounds the outer circumferential portion of the ion exchange membrane 45 in the second electrode assembly 4. Furthermore, the first insulating portion 11 is placed between the ion exchange membrane 35 and the ion exchange membrane 45 so that the first electrode assembly 3 and the second electrode assembly 4 are separated from each other. In addition, the second insulating portion 12 is placed between the ion exchange membrane 45 and the adhesive portion 9 so that the second electrode assembly 4 and the adhesive portion 9 are separated from each other.

In the iontophoresis device 8 shown in Fig. 4, the electric power source device 2, the first electrode assembly 3, and the second electrode assembly 4 are constituted integrally. Therefore, the iontophoresis device 8 can be easily arranged on a mucous membrane. Accordingly, the above-described iontophoresis device can be advantageously used particularly when it is arranged in a narrow space such as an oral cavity.

In a general iontophoresis device, the first electrode assembly is constituted as a working electrode assembly for releasing an ionic drug, and the second electrode assembly is constituted as a non-working electrode assembly (a ground electrode assembly). In some embodiments, however, each of both electrode assemblies as described above can be constituted to release an ionic drug, and the present invention includes such embodiments as well.

A mucous membrane to which such iontophoresis device as described above is applicable is preferably an oral mucous membrane (such as a gum). The iontophoresis device according to the present invention is applicable also to a surface tissue of a living body containing water except a mucous membrane as desired.

A member constituting the adhesive portion is preferably at least one selected from the group consisting of alginic acid, pectin, lower methoxyl pectin, guar gum, gum arabic, cargeenan, methylcellulose, carboxymethylcellulose sodium, xanthan gum, hydroxypropylcellulose, hydroxypropylmethylcellulose, and crystalline cellulose carmellose sodium, and is more preferably at least one selected from the group consisting of carboxymethylcellulose sodium, xanthan gum, hydroxypropylmethylcellulose, and crystalline cellulose carmellose sodium.

Examples of a method of arranging such member constituting the adhesive portion as described above on the end face portion or outer circumferential portion of the first electrode assembly or of the second electrode assembly include: a method involving coating with a member constituting the adhesive portion which contains water or is dry; and a method involving laminating such member by compression.

Each of the first insulating portion and the second insulating portion in the present invention is constituted by a known insulating material such as polystyrene or rubber. Such insulating portion can be arranged on the outer circumferential portion of the first electrode assembly or of the second electrode assembly through, for example, lamination by compression.

Specific examples of an ionic drug applicable to iontophoresis include an ionic drug that can be positively charged and an ionic drug that can be negatively charged. Examples of the ionic drug that can be positively charged include: anesthetic drugs (such as procaine hydrochloride and lidocaine hydrochloride); hemostatic drugs (such as tranexamic acid and ε-aminocaproic acid); antibiotics (such as a tetracycline-based preparation, a kanamycin-based preparation, and a gentamicin-based preparation); and vitamin (such as vitamin B1).

Examples of the ionic drug that can be negatively charged include: vitamin (such as vitamin B2 and vitamin C); adrenal cortex hormones (such as a hydrocortisone-based water-soluble preparation, a dexamethasone-based water-soluble preparation, and a prednisolone-based water-soluble preparation); and antibiotics (such as a penicillin-based water-soluble preparation and a chloramphenicale-based water-soluble preparation).

An ionic drug amount is determined for each individual ionic drug in such a manner that a preset effective blood concentration can be obtained for an effective time period upon application of the drug to a patient, and is set by one skilled in the art in accordance with, for example, the size and thickness of a drug solution holding portion or the like, the area of a drug release surface, a voltage in an electrode device, and an administration time.

In addition, an inactive electrode made of a conductive material such as carbon or platinum can be preferably used as the electrode of the electrode assembly.

The electrolyte solution holding portion can be constituted by a thin film that has the property of holding an electrolyte solution by being impregnated with the electrolyte solution. The thin film can be made of the same material as that used for a drug solution holding portion impregnated with the ionic drug to be described later.

A desired one can be appropriately used as the electrolyte solution depending upon the conditions such as a drug to be applied. However, an electrolyte solution that damages the skin of a living body owing to an electrode reaction should be avoided. An organic acid or a salt thereof present in a metabolic cycle of a living body is preferable as the electrolyte solution in the present invention in terms of harmlessness. For example, lactic acid and fumaric acid are preferable. Specifically, an aqueous solution of 1M of lactic acid and 1M of sodium fumarate (1 : 1) is preferable. Such electrolyte solution is preferable because: it has high solubility with respect to water and passes a current well; and in the case where a current is allowed to flow at a constant level, the electric resistance is low and a change in pH is relatively small in an electric power source device.

A cation exchange membrane and an anion exchange membrane are preferably used together as ion exchange membranes to be used for an electrode assembly. Preferable examples of the cation exchange membrane include NEOSEPTAs (CM-1, CM-2, CMX, CMS, CMB, and CLE04-2) manufactured by Tokuyama Co., Ltd. Preferable examples of the anion exchange membrane include NEOSEPTAs (AM-1, AM-3, AMX, AHA, ACH, ACS, ALE04-2, and AIP-21) manufactured by Tokuyama Co., Ltd. Other preferable examples include: a cation exchange membrane that includes a porous film having cavities a part or whole of which are filled with an ion exchange resin having a cation exchange function; and an anion exchange membrane that includes a porous film having cavities a part or whole of which are filled with an ion exchange resin having an anion exchange function.

The above-mentioned ion exchange resins can be fluorine-based ones that include a perfluorocarbon skeleton having an ion exchange group and hydrocarbon-based ones that include a nonfluorinated resin as a skeleton. From the viewpoint of convenience of production process, hydrocarbon-based ion exchange resins are preferably used. The filling rate of the above-mentioned porous film with the ion exchange resin, which varies depending on the porosity of the porous film, can be, for example, 5 to 95 % by mass, and is preferably 10 to 90 % by mass, or more preferably 20 to 60% by mass.

The ion exchange group in the above-mentioned ion exchange resin is not particularly limited so far as it is a functional group that generates a group having negative or positive charge in aqueous solutions. Such functional group may be present in the form of a free acid or a salt. Examples of a cation exchange group include a sulfonic group, a carboxylic acid group, and a phosphonic acid group. Of those, a sulfonic group is preferable. Examples of a counter cation for the cation exchange group include: alkali cations such as a sodium ion and a potassium ion; and ammonium ions. Examples of an anion exchange group include a primary amino group, a secondary amino group, a tertiary amino group, a quaternary ammonium group, a pyridyl group, an imidazole group, a quaternary pyridium group, and a quaternary imidazolium group. Of those, a quaternary ammonium group or a quaternary pyridium group is preferable. Examples of a counter cation for the anion exchange group include: halogen ions such as a chlorine ion; and hydroxy ions.

The above-mentioned porous film is not particularly limited and any porous film can be used as far as it is in the form of a film or sheet that has a large number of pores communicating both sides thereof. To satisfy both of high strength and flexibility, it is preferable that the porous film be made of a thermoplastic resin. Examples of the thermoplastic resin constituting the porous film include: polyolefin resins such as homopolymers or copolymers of α-olefins such as ethylene, propylene, 1-butene, 1-pentene, 1-hexene, 3-methyl-1-butene, 4-methyl-1-pentene, and 5-methyl-1-heptene; vinyl chloride-based resins such as polyvinyl chloride, vinyl chloride-vinyl acetate copolymers, vinyl chloride-vinylidene chloride copolymers, and vinyl chloride-olefin copolymers; fluorine-based resins such as polytetrafluoroethylene, polychlorotrifluoroethylene, polyvinylidene fluoride, tetrafluoroethylene-hexafluoropropylene copolymers, tetrafluoroethylene-perfluoroalkyl vinylether copolymers, and tetrafluoroethylene-ethylene copolymers; polyamide resins such as nylon 66; and polyimide resins. Of those, polyolefin resins are preferable in consideration of, for example, mechanical strength, flexibility, chemical stability, and chemical resistance. Of those, polyethylene or polypropylene is more preferable, and polyethylene is still more preferable.

The properties of the above-mentioned porous film made of the thermoplastic resin are not particularly limited. However, the mean pore size is preferably 0.005 to 5.0 µm, more preferably 0.01 to 2.0 µm, or still more preferably 0.02 to 0.2 µm in consideration of the formation of an ion exchange membrane that is thin and has excellent strength and low electric resistance. The above-mentioned mean pore size as used herein means a mean flow pore size measured in conformance with the bubble point method (JIS K3832-1990). Similarly, the porosity of the porous film is preferably 20 to 95 %, more preferably 30 to 90 %, or still more preferably 30 to 60 %. In consideration of the thickness of an ion exchange membrane to be finally formed, the thickness of the porous film is preferably 5 to 140 µm, more preferably 10 to 130 µm, or still more preferably 15 to 55 µm. Usually, an anion exchange membrane or a cation exchange membrane formed of such porous film generally has the same thickness as that of the porous film or up to about 20 µm larger than the thickness of the porous film.

Furthermore, the drug solution holding portion is constituted by a thin film that holds a drug or the like by being impregnated with the drug or the like. It is important for such thin film to have a sufficient ability of holding a drug or the like by being impregnated with the drug or the like, and a sufficient ability (ion transferability, ion conductivity) of moving an ionized drug impregnated into and held by the thin film to the skin side under predetermined electric field conditions. Examples of a material that brings together good property of holding a drug by being impregnated with the drug and good ion conductivity include hydrogel forms of acrylic resins (acrylic hydrogel film), a segmented polyurethane-based gel film, and an ion-conductive porous sheet for forming a gel-like solid electrolyte (such as a porous polymer disclosed in JP 11-273452 A using, as a base, an acrylonitrile copolymer containing 50 mol% or more, preferably 70 to 98 mol% or more of acrylonitrile and having a porosity of 20 to 80%). When such drug solution holding portion as described above is impregnated with a drug, an impregnation rate (defined by 100x(W-D)/D (%) where D indicates a dry weight and W indicates a weight after impregnation) is preferably 30 to 40%.

The following conditions are adopted as preferred energizing conditions in the iontophoresis device as described above.
(1) Constant current condition, specifically, 0.01 to 0.7 mA/cm², preferably 0.1 to 0.5 mA/cm²
(2) Safe voltage condition that realizes the above constant current, specifically, 50 V or less, preferably 30 V or less

WO 03/037425 A1 according to the applicant of the present invention describes details about the above-described respective components, and the contents described in the document are also included in the present invention.

## Claims

1. An iontophoresis device comprising:
an electric power source device;
a first electrode assembly for administering an ionic drug to a living body through a mucous membrane by releasing the ionic drug by iontophoresis, the first electrode assembly being connected to the electric power source device; and
a second electrode assembly as a counter electrode of the first electrode assembly,
wherein the first electrode assembly comprises an adhesive portion for bringing the mucous membrane and the first electrode assembly into contact with each other, the adhesive portion being placed on at least a part of an end face portion of the first electrode assembly, and
the adhesive portion is composed of a member exhibiting adhesiveness owing to absorption of an aqueous medium.

2. The iontophoresis device according to claim 1, wherein the first electrode assembly comprises:
an electrode connected to an electric power source device having the same polarity as that of a drug component of the ionic drug;
an electrolyte solution holding portion impregnated with the electrolyte solution, the electrolyte solution holding portion being placed adjacent to the electrode;
an ion exchange membrane which is selectively permeable to an ion having a polarity opposite to that of a charged ion of the ionic drug, the ion exchange membrane being placed adjacent to the electrolyte solution holding portion;
a drug solution holding portion impregnated with the ionic drug, the drug solution holding portion being placed adjacent to the ion exchange membrane;
an ion exchange membrane which is selectively permeable to an ion having the same polarity as that of the charged ion of the ionic drug, the ion exchange membrane being placed adjacent to the drug solution holding portion; and
the adhesive portion for bringing the mucous membrane and the first electrode assembly into contact with each other, the adhesive portion being placed on at least a part of the end face portion of the first electrode assembly, and the adhesive portion is composed of the member exhibiting adhesiveness owing to absorption of the aqueous medium.

3. The iontophoresis device according to claim 2, wherein the adhesive portion for bringing the mucous membrane and the first electrode assembly into contact with each other is placed on an outer circumferential portion of the ion exchange membrane which is selectively permeable to an ion having the same polarity as that of the charged ion of the ionic drug.

4. The iontophoresis device according to claim 1, wherein the second electrode assembly comprises an adhesive portion for bringing the mucous membrane and the second electrode assembly into contact with each other, the adhesive portion being placed on at least a part of an end face portion of the second electrode assembly, and the adhesive portion is composed of a member exhibiting adhesiveness owing to absorption of the aqueous medium.

5. The iontophoresis device according to claim 4, wherein the second electrode assembly comprises:
an electrode having a polarity opposite to that of the electrode in the first electrode assembly;
an electrolyte solution holding portion impregnated with the electrolyte solution, the electrolyte solution holding portion being placed adjacent to the electrode;
an ion exchange membrane which is selectively permeable to an ion having a polarity opposite to that of a charged ion of the ionic drug, the ion exchange membrane being placed adjacent to the electrolyte solution holding portion; and
the adhesive portion for bringing the mucous membrane and the second electrode assembly into contact with each other, the adhesive portion being placed on at least a part of the end face portion of the second electrode assembly, and the adhesive portion is composed of the member exhibiting adhesiveness owing to absorption of the aqueous medium.

6. The iontophoresis device according to claim 4, wherein the second electrode assembly comprises:
an electrode having a polarity opposite to that of the electrode in the first electrode assembly;
an electrolyte solution holding portion impregnated with the electrolyte solution, the electrolyte solution holding portion being placed adjacent to the electrode;
an ion exchange membrane which is selectively permeable to an ion having the same polarity as that of a charged ion of the ionic drug, the ion exchange membrane being placed adjacent to the electrolyte solution holding portion;
an electrolyte solution holding portion impregnated with the electrolyte solution, the electrolyte solution holding portion being placed adjacent to the ion exchange membrane;
an ion exchange membrane which is selectively permeable to an ion having a polarity opposite to that of the charged ion of the ionic drug, the ion exchange membrane being placed adjacent to the electrolyte solution holding portion; and
the adhesive portion for bringing the mucous membrane and the second electrode assembly into contact with each other, the adhesive portion being placed on at least a part of the end face portion of the second electrode assembly, and the adhesive portion is composed of the member exhibiting adhesiveness owing to absorption of the aqueous medium.

7. The iontophoresis device according to claim 5 or 6, wherein the adhesive portion for bringing the mucous membrane and the second electrode assembly into contact with each other is placed on an outer circumferential portion of the ion exchange membrane which is selectively permeable to an ion having a polarity opposite to that of the charged ion of the ionic drug.

8. An iontophoresis device comprising:
an electric power source device;
a first electrode assembly for administering an ionic drug to a living body through the mucous membrane by releasing the ionic drug by iontophoresis, the first electrode assembly being connected to the electric power source device; and
a second electrode assembly as a counter electrode of the first electrode assembly,
wherein the second electrode assembly surrounds an outer circumferential portion of the first electrode assembly,
the iontophoresis device comprises an adhesive portion for bringing the mucous membrane and the iontophoresis device into contact with each other, the adhesive portion surrounding an outer circumferential portion of the second electrode assembly, and
the adhesive portion is composed of a member exhibiting adhesiveness owing to absorption of an aqueous medium.

9. The iontophoresis device according to claim 8, wherein the first electrode assembly comprises:
an electrode connected to an electric power source device having the same polarity as that of a drug component of the ionic drug;
an electrolyte solution holding portion impregnated with the electrolyte solution, the electrolyte solution holding portion being placed adjacent to the electrode;
an ion exchange membrane which is selectively permeable to an ion having a polarity opposite to that of a charged ion of the ionic drug, the ion exchange membrane being placed adjacent to the electrolyte solution holding portion;
a drug solution holding portion impregnated with the ionic drug, the drug solution holding portion being placed adjacent to the ion exchange membrane; and
an ion exchange membrane which is selectively permeable to an ion having the same polarity as that of the charged ion of the ionic drug, the ion exchange membrane being placed adjacent to the drug solution holding portion.

10. The iontophoresis device according to claim 8, wherein the second electrode assembly comprises:
an electrode having a polarity opposite to that of the electrode in the first electrode assembly;
an electrolyte solution holding portion impregnated with the electrolyte solution, the electrolyte solution holding portion being placed adjacent to the electrode; and
an ion exchange membrane which is selectively permeable to an ion having a polarity opposite to that of the charged ion of the ionic drug, the ion exchange membrane being placed adjacent to the electrolyte solution holding portion.

11. The iontophoresis device according to claim 8, wherein the second electrode assembly comprises:
an electrode having a polarity opposite to that of the electrode in the first electrode assembly;
an electrolyte solution holding portion impregnated with the electrolyte solution, the electrolyte solution holding portion being placed adjacent to the electrode;
an ion exchange membrane which is selectively permeable to an ion having the same polarity as that of a charged ion of the ionic drug, the ion exchange membrane being placed adjacent to the electrolyte solution holding portion;
an electrolyte solution holding portion impregnated with the electrolyte solution, the electrolyte solution holding portion being placed adjacent to the ion exchange membrane; and
an ion exchange membrane which is selectively permeable to an ion having a polarity opposite to that of the charged ion of the ionic drug, the ion exchange membrane being placed adjacent to the electrolyte solution holding portion.

12. The iontophoresis device according to claim 8, further comprising a first insulating portion placed between the first electrode assembly and the second electrode assembly.

13. The iontophoresis device according to claim 8, further comprising a second insulating portion placed between the second electrode assembly and the adhesive portion.

14. The iontophoresis device according to any one of claims 1 to 13, wherein the member exhibiting adhesiveness owing to adsorption of the aqueous medium comprises at least one selected from the group consisting of alginic acid, pectin, low-methoxyl pectin, guar gum, gum arabic, carageenan, methylcellulose, sodium carboxymethyl cellulose, xanthan gum, hydroxypropylcellulose, hydroxypropylmethylcellulose, and crystalline cellulose carmellose sodium.

15. The iontophoresis device according to any one of claims 1 to 13, wherein the member exhibiting adhesiveness owing to adsorption of the aqueous medium comprises at least one selected from the group consisting of carboxymethylcellulose, xanthan gum, hydroxypropylmethylcellulose, and crystalline cellulose carmellose sodium.

16. The iontophoresis device according to any one of claims 1 to 15,wherein the mucous membrane is an oral mucous membrane.

17. A method of operating the iontophoresis device according to any one of claims 1 to 16, comprising at least:
placing the first electrode assembly and the second electrode assembly on the mucous membrane;
energizing the iontophoresis device with the electric power source device; and
allowing the first electrode assembly to release the ionic drug.
